(19) Europäisches Patentamt
European Patent Office
Office européen des brevets

(11) Veröffentlichungsnummer: **0 309 914 A2**

(12) # EUROPÄISCHE PATENTANMELDUNG

(21) Anmeldenummer: **88115570.9**

(22) Anmeldetag: **22.09.88**

(51) Int. Cl.4: **C07D 207/06 , C07D 207/08 , C07D 405/04 , C07F 7/08 , A01N 43/36**

(30) Priorität: **30.09.87 DE 3732910**

(43) Veröffentlichungstag der Anmeldung: **05.04.89 Patentblatt 89/14**

(84) Benannte Vertragsstaaten: **AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Zipplies, Matthias, Dr.**
**Kastanienweg 1**
**D-6945 Hirschberg(DE)**
Erfinder: **Sauter, Hubert, Dr.**
**Neckarpromenade 20**
**D-6800 Mannheim 1(DE)**
Erfinder: **Himmele, Walter, Dr.**
**Eichenwweg 14**
**D-6909 Walldorf(DE)**
Erfinder: **Ammermann, Eberhard, Dr.**
**Sachsenstrasse 3**
**D-6700 Ludwigshafen(DE)**
Erfinder: **Pommer, Ernst-Heinrich, Dr.**
**Berliner Platz 7**
**D-6703 Limburgerhof(DE)**

(54) **Fungizide N-substituierte 3-Alkyl-4-aryl-pyrrolidin-Derivate.**

(57) 3-Alkyl-4-aryl-pyrrolidin-Derivate der Formel

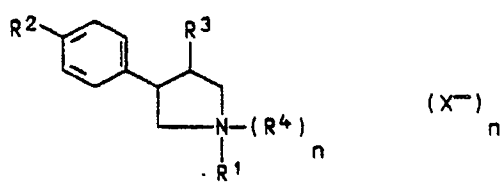

I

in der

$R^1$ Alkenyl, Alkinyl, Cycloalkyl, Cycloalkenyl, Alkyl-cycloalkyl, Cycloalkyl-alkyl, Alkyl-cycloalkyl-alkyl, Cycloalkenyl-alkyl, Alkyl-cycloalkenyl, Bicycloalkyl, Bicycloalkylalkyl, Alkyl-bicycloalkyl, Heterocycloalkyl, Heterocycloalkylmethyl, Aryl, Arylalkyl, Alkylaryl, Alkyl-aryl-alkyl,

$R^2$ Alkyl, Alkoxy,

$R^3$ Alkyl

$R^4$ Alkyl, Alkenyl, Alkinyl und Phenylalkyl,

$X^-$ ein für Pflanzen verträgliches Anion bedeutet,

n den Wert 0 oder 1 hat,

EP 0 309 914 A2

Xerox Copy Centre

deren für Pflanzen verträgliche Salze und Fungizide, die diese Verbindungen enthalten.

# Fungizide N-substituierte 3-Alkyl-4-aryl-pyrrolidin-Derivate

Die vorliegende Erfindung betrifft neue N-substituierte 3-Alkyl-4-aryl-pyrrolidine, Verfahren zu deren Herstellung, deren Verwendung als Fungizide, fungizide Mittel, die die neuen Wirkstoffe enthalten, Verfahren zur Herstellung solcher fungizider Mittel sowie Verfahren zur Bekämpfung von Pilzen mit diesen Wirkstoffen.

Aus DE 2 727 482 ist ein Arylalkylpyrrolidinderivat der folgenden Formel als Verbindung mit fungizider Wirkung bekannt.

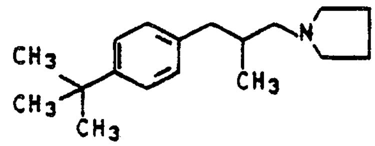

Seine fungizide Wirkung ist jedoch unbefriedigend.

Es wurde nun gefunden, daß Verbindungen der Formel I

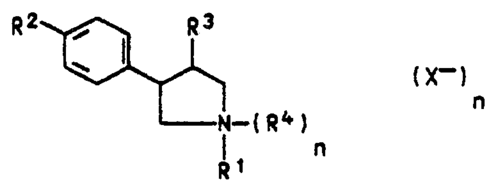

I

in der

$R^1$ gegebenenfalls durch Hydroxy, Halogen, $C_1$-$C_5$-Alkoxy oder $C_3$-$C_9$-Trialkylsilyl substituiertes, $C_2$-$C_{20}$-Alkyl, $C_3$-$C_{20}$-Alkenyl, $C_3$-$C_{20}$-Alkinyl, gegebenenfalls substituiertes $C_4$-$C_{12}$ Cycloalkyl, $C_4$-$C_{12}$-Cycloalkenyl, gegebenenfalls substituiertes $C_4$-$C_{20}$-Alkyl-cycloalkyl, $C_4$-$C_{20}$-Cycloalkyl-alkyl, $C_4$-$C_{20}$-Alkylcycloalkyl-alkyl, $C_4$-$C_{20}$-Cycloalkenyl-alkyl, gegebenenfalls substituiertes $C_9$-$C_{11}$-Bicycloalkyl, $C_{10}$-$C_{15}$-Bicycloalkylalkyl, gegebenenfalls substituiertes $C_{10}$-$C_{15}$-Alkyl-bicycloalkyl, 5 bis 7-gliedriges Heterocycloalkyl mit 1 oder 2 Heteroatomen aus der Gruppe Sauerstoff und/oder Schwefel, 5 bis 7-gliedriges Heterocycloalkylmethyl mit 1 oder 2 Heteroatomen aus der Gruppe Sauerstoff und/oder Schwefel, $C_1$-$C_8$-Alkyl-substituiertes 5 bis 7-gliedriges Heterocycloalkyl oder Heterocycloalkylmethyl mit 1 oder 2 Heteroatomen aus der Gruppe Sauerstoff und/oder Schwefel, gegebenenfalls substituiertes Aryl, gegebenenfalls substituiertes $C_7$-$C_{20}$-Arylalkyl, $C_7$-$C_{20}$-Alkylaryl, $C_8$-$C_{20}$-Alkyl-aryl-alkyl,

$R^2$ $C_3$-$C_{10}$-Alkyl, $C_3$-$C_8$-Alkoxy

$R^3$ $C_1$-$C_4$-Alkyl

$R^4$ $C_1$-$C_5$-Alkyl, $C_3$-$C_5$-Alkenyl, $C_3$-$C_5$-Alkinyl, $C_7$-$C_8$-Phenylalkyl,

$X^-$ ein für Pflanzen verträgliches Anion bedeutet und

n den Wert 0 oder 1 hat

und deren für Pflanzen verträgliche Salze bei guter Pflanzenverträglichkeit hervorragend fungizid wirksam sind.

Unter Salzen sind Salze mit für Pflanzen verträglichen Anionen $X^-$ von beliebigen anorganischen und organischen Säuren zu verstehen, z.B. Chlorwasserstoff, Fluorwasserstoff, Iodwasserstoff, Bromwasserstoff, Schwefelsäure, Phosphorsäure, Dodecylbenzolsulfonsäure, $C_1$-$C_{16}$-Alkylcarbonsäuren, Ameisensäure, Essigsäure, Propionsäure, Palmitinsäure, Perfluorheptansäure, Oxalsäure, Malonsäure, Benzoesäure, Äpfelsäure, Dodecylschwefelsäure, Glycerin-2-phosphorsäure, Methylschwefelsäure, Methansulfonsäure, p-Toluolsulfonsäure, Salpetersäure und Salze wie Hydrogensulfate oder Dihydrogenphosphate.

Die neuen N-substituierten 3-Alkyl-4-aryl-pyrrolidine der Formel I und deren Salze enthalten chirale Zentren. Sie werden im allgemeinen als Racemate oder gegebenenfalls als Diastereomerengemische erhalten. Diastereomerenreine Verbindungen lassen sich bei einigen der neuen Verbindungen beispielsweise durch Destillation, Säulenchromatographie oder aufgrund von Löslichkeitsunterschieden isolieren. Enantiomerenreine Verbindungen lassen sich z. B. durch Racematspaltung mit einem chiralen Hilfsreagenz nach bekannten Methoden beispielsweise über diastereomere Salze erhalten. Für die Anwendung der neuen N-substituierten 3-Alkyl-4-aryl-pyrrolidine als Fungizide sind sowohl die Diastereomeren bzw. die Enantiomeren als auch deren bei der Synthese anfallenden Stereoisomerengemische geeignet. Sie alle werden von der Erfindung umfaßt.

$R^1$ bedeutet beispielsweise

gerades oder verzweigtes $C_2$-$C_{20}$ Alkyl, besonders $C_3$-$C_{13}$-Alkyl, wie beispielsweise Propyl, Isopropyl, Butyl, Isobutyl, But-2-yl, tert.-Butyl, Pentyl, Pent-2-yl, Pent-3-yl, 1,2-Dimethyl-propyl, 2,2-Dimethyl-propyl, Hexyl, Hex-2-yl, Hex-3-yl, 2,3,3-Trimethyl-but-2-yl, 4-Methyl-pent-2-yl, 4-Methyl-pentyl, 3,3-Dimethylbutyl, Heptyl, Hept-2-yl, Hept-3-yl, Hept-4-yl, Diisopropyl-methyl, 1,4-Dimethyl-pentyl, 4,4-Dimethyl-pentyl, Octyl, 2-Methyl-hept-3-yl, 5-Methyl-hept-3-yl, Oct-2-yl, Oct-3-yl, Oct-4-yl, 5,5-Dimethyl-hexyl, 2,4,4-Trimethyl-pentyl, 6-Methyl-hept-2-yl, Nonyl, Non-2-yl, Non-3-yl, Non-4-yl, Non-5-yl, 2,5,5-Trimethyl-hexyl, 2,6-Di methyl-hept-4-yl, 3,5,5-Trimethyl-hexyl, Decyl, Dec-2-yl, Dec-3-yl, Dec-4-yl, 2,3,5,5-Tetramethyl-hexyl, Undecyl, Dodecyl, Tridecyl, 1,5,9-Trimethyl-decyl, Tetradecyl,

$C_2$-$C_{20}$-Hydroxyalkyl, besonders $C_2$-$C_8$-Hydroxyalkyl, wie beipielsweise Hydroxyethyl, Hydroxypropyl, Hydroxybutyl, Hydroxypentyl, Hydroxyhexyl, Hydroxyheptyl, 1-Hydroxy-but-2-yl, 2-Hydroxy-but-3-yl,

$C_2$-$C_{20}$-Halogenalkyl mit 1-3 Halogenatomen wie Chlor, Brom, Fluor, besonders $C_2$-$C_{10}$-Halogenalkyl mit 1-3 Halogenatomen wie Chlor, Brom, Fluor, wie beispielsweise 3-Chlorpropyl, 6-Chlorhexyl, Trifluorethyl, Trichlorethyl, 5-Chlor-pent-2-yl, 3-Chlor-but-2-yl, 3,3-Dichlor-prop-2-yl,

$C_1$-$C_5$-alkoxy-$C_2$-$C_{20}$-Alkyl, besonders $C_1$-$C_4$-Alkoxy-$C_2$-$C_{10}$-Alkyl, wie beispielsweise Methoxyethyl, Ethoxyethyl, tert-Butoxyethyl, 3-tert.-Butoxy-propyl, 4-tert.-Butoxy-butyl, Methoxypropyl, Methoxybutyl, Methoxypentyl, Methoxyhexyl, Ethoxyhexyl, 3-Methoxy-prop-2-yl,

$C_3$-$C_9$ Trialkylsilyl-$C_2$-$C_{20}$-Alkyl, besonders $C_3$-$C_6$-Trialkylsilyl, $C_2$-$C_{10}$-Alkyl, wie beispielsweise Trimethylsilyl-ethyl, Trimethylsilyl-propyl, Trimtehylsilyl-butyl, Trimethylsilyl-pentyl, 6-Trimethylsilyl-hexyl,

$C_3$-$C_{20}$-Alkenyl, besonders $C_3$-$C_{14}$-Alkenyl, wie beispielsweise Allyl, Methallyl, Dimethylallyl, Hexenyl, 1,5,9-Trimethyl-decadienyl,

$C_3$-$C_{20}$-Alkinyl, besonders $C_3$-$C_6$-Alkinyl, wie beispielsweise Propargyl, 4,4-Dimethyl-2-butin-1-yl,

$C_4$-$C_{12}$-Cycloalkyl, wie beispielsweise Cyclobutyl, Cyclopentyl, Cyclohexyl, Cycloheptyl, Cycloedodecyl,

$C_4$-$C_{12}$-Hydroxycycloalkyl, wie beispielsweise 4-Hydroxycyclohexyl,

$C_1$-$C_5$-Alkoxy-$C_4$-$C_{12}$-Cycloalkyl, wie 4-Methoxycyclohexyl, 4-tert.-Butoxycyclohexyl,

$C_3$-$C_9$-Trialkylsilyl-$C_4$-$C_{12}$-Cycloalkyl, wie beispielsweise 4-Trimethylsilylcyclohexyl,

$C_4$-$C_{12}$-Cycloalkenyl, wie beispielsweise Cyclopentenyl, Cyclohexenyl, Cycloheptenyl,

$C_4$-$C_{20}$-Alkylcycloalkyl, wie beispielsweise, $C_1$-$C_8$-Alkyl-$C_4$-$C_8$-Cycloalkyl, 4-Methylcyclohexyl, 3-Methyl-Cyclohexyl, 3,3-Dimethylcyclohexyl, 3,3,5-Trimethylcyclohexyl, 4-Isopropyl-cyclohexyl, 4-tert.-Butyl-cyclohexyl, 4(2-Methyl-but-2-yl)cyclohexyl, 4(2,4,4-Trimethyl-pent-2-yl)cyclohexyl,

$C_4$-$C_{10}$-Alkyl-hydroxycycloalkyl, wie beispielsweise $C_1$-$C_4$-Alkyl-hydroxy-$C_4$-$C_8$-cycloalkyl, 4-Hydroxy-3,6-dimethyl-cyclohexyl, 4-Hydroxy-3,3-dimethyl-cyclohexyl, 4-Hydroxy-3,3,5-trimethylcyclohexyl,

$C_5$-$C_{20}$-Cycloalkyl-alkyl, wie beispielsweise $C_4$-$C_8$-Cycloalkyl-$C_1$-$C_4$-alkyl, Cyclohexylmethyl, Cyclohexylethyl, Cyclohexylpropyl,

$C_4$-$C_{20}$-Alkylcycloalkenyl, wie beispielsweise 4-Isopropyl-cyclohexenyl, 4-tert.-Butyl-cyclohexenyl, $C_9$-$C_{11}$-Bicycloalkyl, wie beispielsweise [4.3.0.]Bicyclononyl, Decalyl, $C_9$-$C_{11}$-Hydroxybicycloalkyl, wie beispielsweise 6-Hydroxy-2-decalyl, 7-Hydroxy-2-decalyl,

$C_{10}$-$C_{15}$-Alkyl-bicycloalkyl, wie beipielsweise 9-Methyl-2-decalyl, 5,9-Dimethyl-2-decalyl, 5,5,9-Trimethyl-2-decalyl,

$C_{10}$-$C_{15}$-Alkyl-hydroxybicycloalkyl, wie beispielsweise 6-Hydroxy-9-methyl-2-decalyl, 6-Hydroxy-5,9-dimethyl-2-decalyl, 6-Hydroxy-5,5,9-Trimethyl-2-decalyl,

5 bis 7-gliedriges Heterocycloalkyl mit 1 oder 2 Heteroatomen aus der Gruppe Sauerstoff oder Schwefel, wie beispielsweise Tetrahydropyranyl, Tetrahydrothiopyranyl, Dioxanyl,

5 bis 7 gliedriges Heterocycloalkylmethyl mit 1 oder 2 Heteroatomen aus der Gruppe Sauerstoff oder Schwefel, wie beispielsweise Tetrahydropyranylmethyl, Dioxanylmethyl,

$C_1$-$C_8$-Alkyl-substituiertes 5 bis 7-gliedriges Heterocycloalkyl oder Heterocycloalkylmethyl mit 1 oder 2 Heteroatomen aus der Gruppe Sauerstoff oder Schwefel, wie beispielsweise 3,5-Diethyl-dioxan-2-yl-methyl, 3,6-Diethyl-dioxan-2-yl-methyl, 3,5-Dimethyl-dioxan-2-yl-methyl,

gegebenenfalls durch Hydroxy, 1-3 Halogenatomen wie Chlor, Brom, Fluor, $C_2$-$C_5$-Alkoxy, $C_3$-$C_9$-Trialkylsilyl substituiertes Aryl, z.B. Phenyl, 4-Hydroxyphenyl, 4-Chlorphenyl, 2,4-Dichlorphenyl, 4-tert.-Butoxyphenyl, 4-Trimethylsilylphenyl,

Gegebenenfalls durch Hydroxy, 1-3 Halogenatome wie Chlor, Brom, Fluor, $C_1$-$C_5$-Alkoxy, $C_3$-$C_9$-Trialkylsilyl substituertes $C_7$-$C_{20}$-Arylalkyl, wie beispielsweise Benzyl, 4-Chlorbenzyl, 2,4-Dichlorbenzyl, 4-Brombenzyl, 4-tert.-Butoxybenzyl, 4-Trimethylsilylbenzyl, 4-Phenyl-4-methyl-pentyl,

$C_7$-$C_{20}$ Alkylaryl, wie beispielweise 4-tert.-Butyl-phenyl,

$C_8$-$C_{20}$-Alkylarylalkyl, wie beispielsweise 4-tert.-Butyl-benzyl, 4-tert.-Butyl-phenethyl, 4-tert.-Butyl-phenylpropyl, 3(4-tert.-Butyl-phenyl)-2-methyl-propyl.

$R^2$ bedeutet beispielsweise

Verzweigtes oder unverzweigtes $C_3$-$C_{10}$-Alkyl bzw. $C_3$-$C_8$-Alkoxy, wie beispielsweise Propyl, Isopropyl, sec.-Butyl, tert.-Butyl, 2-Methyl-but-2-yl, 2,4,4-Trimethyl-pent-2-yl, Propyloxy, Butoxy, tert.-Butoxy.

$R^3$ bedeutet beispielsweise

$C_1$-$C_4$-Alkyl, wie beispielsweise Methyl, Ethyl, Propyl, Isopropyl, Butyl,

$R^4$ bedeutet beispielsweise

$C_1$-$C_5$-Alkyl, wie beispielsweise Methyl, Ethyl, Propyl, $C_3$-$C_5$-Alkenyl, wie beispielsweise Allyl, Methallyl, $C_3$-$C_5$-Alkinyl, wie beispielsweise Propargyl, Butinyl, $C_7$-$C_8$-Phenylalkyl, wie beispielsweise Benzyl.

Verbindungen der Formel I können beispielsweise wie folgt hergestellt werden.

- N-Alkylierung von Pyrrolidinen
- im Fall, daß $R^2$ = Alkyl ist, durch Friedel-Crafts-Alkylierung von N-alkylierten Phenyl-pyrrolidinen
- den Heteroring aufbauende Reaktionen, welche von geeigneten Vorstufen ausgehen und anschließender Reduktion.

Die Verfahren werden im folgenden erklärt:

Einführung des Restes $R^1$.

a) Umsetzung eines Pyrrolidinderivates II mit einer Verbindung $R^1$-X unter basischen Bedingungen.

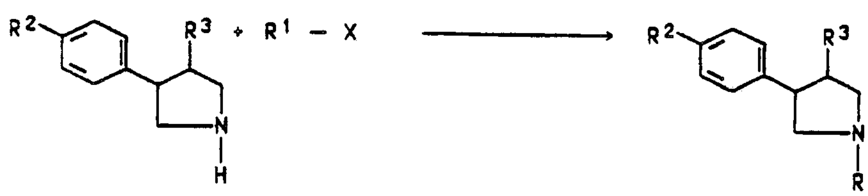

(II) (I)

Als Reste X seien beispielsweise genannt, Chlor, Brom, Jod, die Methan-, Benzol-, p-Toluolsulfonylgruppe.

Die Reaktion wird bei 40-200 °C, gegebenenfalls in Gegenwart eines inerten Lösungsmittels durchgeführt. Als Basen werden anorganische Basen bevorzugt, beispielsweise Kalium-, Natrium- und Lithrumhydroxid, Natriumhydrid, Kalium- und Natriumcarbonat. Geeignet sind auch organische Basen wie Triethylamin, Dicyclohexylamin und Diisopropylamin. Die Reaktion läßt sich auch mit einem Überschuß des Pyrrolidinderivates II durchführen.

b) Umsetzung einer Carbonylverbindung III,

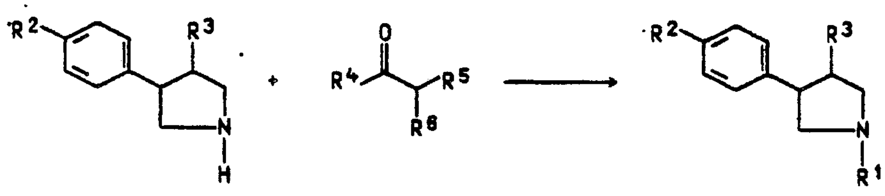

(II) (III) (I)

wobei die Reste $R^4$ bis $R^6$ so definiert sind, daß der Rest

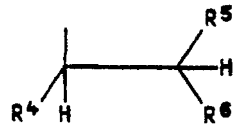

in seiner Gesamtheit dem Rest R[1] entspricht, mit einem Pyrrolidinderivat II unter gleichzeitiger oder anschließender Reduktion bzw. Hydrierung.

$b_1$) Bei der direkten Methode zur Herstellung der Verbindung I wird ein Gemisch aus II und III in Gegenwart eines Lösungsmittels, beispielsweise Methanol, Ethanol, Propanol, Isopropanol, die bis zu 25 % (Vol.) Wasser enthalten können, mit Natriumcyanborhydrid oder Natriumborhydrid, gegebenenfalls in Gegenwart eines Metallsalzes wie Zink(II)chlorid, Cadmium(II)chlorid oder Magnesium(II)chlorid, bei 0-100 °C, vorzugsweise 20-80 °C oder aber in Gegenwart eines Lösungmittels wie beispielsweise Methanol, Ethanol, Tetrahydrofuran oder Toluol und eines Hydrierkatalysators wie z. B Raney-Nickel, Platin-IV-oxid, $Ru_2O_3$ oder Palladium auf Kohle im Autoklaven bei 100-150 °C mit Wasserstoff bis Druckkonstanz umgesetzt.

$b_2$) Bei der Zweistufen-Reaktion wird aus den Verbindungen II und III ein Enamin in an sich bekannter Weise unter wasserentziehenden Bedingungen hergestellt und anschließend mit einem Edelmetallkatalysator wie Raney-Nickel, Raney-Cobalt, $PtO_2$ oder $Ru_2O_3$, vorzugsweise Palladium auf Kohle, mit Wasserstoff hydriert, bzw. mit Ameisensäure nach Leukart-Wallach reduziert.

c) Für den Fall, daß $R^2 = C_3$-$C_{10}$-Alkyl, läßt sich $R^2$ in ein Phenylpyrrolidin-Derivat der Struktur IV

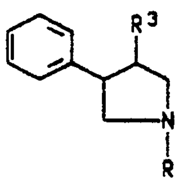

(IV)

einführen, indem man dieses säurekatalysiert mit einem Alken der Struktur Va,

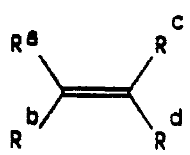

(Va)

in welchem die Reste $R^a$-$R^d$ so definiert sind, daß der Rest Vb in seiner Gesamtheit

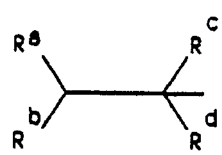

(Vb)

dem Rest $R^2$ entspricht, bzw. mit einem Alkylhalogenid $R^2$-X umsetzt. Man verwendet Mineralsäuren, wie beispielsweise Schwefelsäure, Salzsäure, HF, Bromwasserstoffsäure, oder auch Lewis Säuren wie $AlCl_3$ oder $SnCl_4$ und führt die Reaktion unter Eiskühlung z.B. bei -10 bis +20 °C durch.

d) Ringaufbauende Reaktionen

Die Verbindungen I lassen sich auch durch Umsetzungen von primären Aminen der Formel $R^1$-$NH_2$ mit

einer Verbindung VIa, VIb, VIc, VId und anschließender Reduktion der Carbonylgruppe(n) herstellen.

Bei der Umsetzung von Dicarbonsäuren, Bernsteinsäureanhydriden und 5-Ringlactonen wird das Reaktionswasser entweder am Wasserabscheider mit einem geeigneten Schleppmittel oder in Gegenwart eines wasserbindenden Mittels aus dem Gleichgewicht entfernt. Als Lösungsmittel

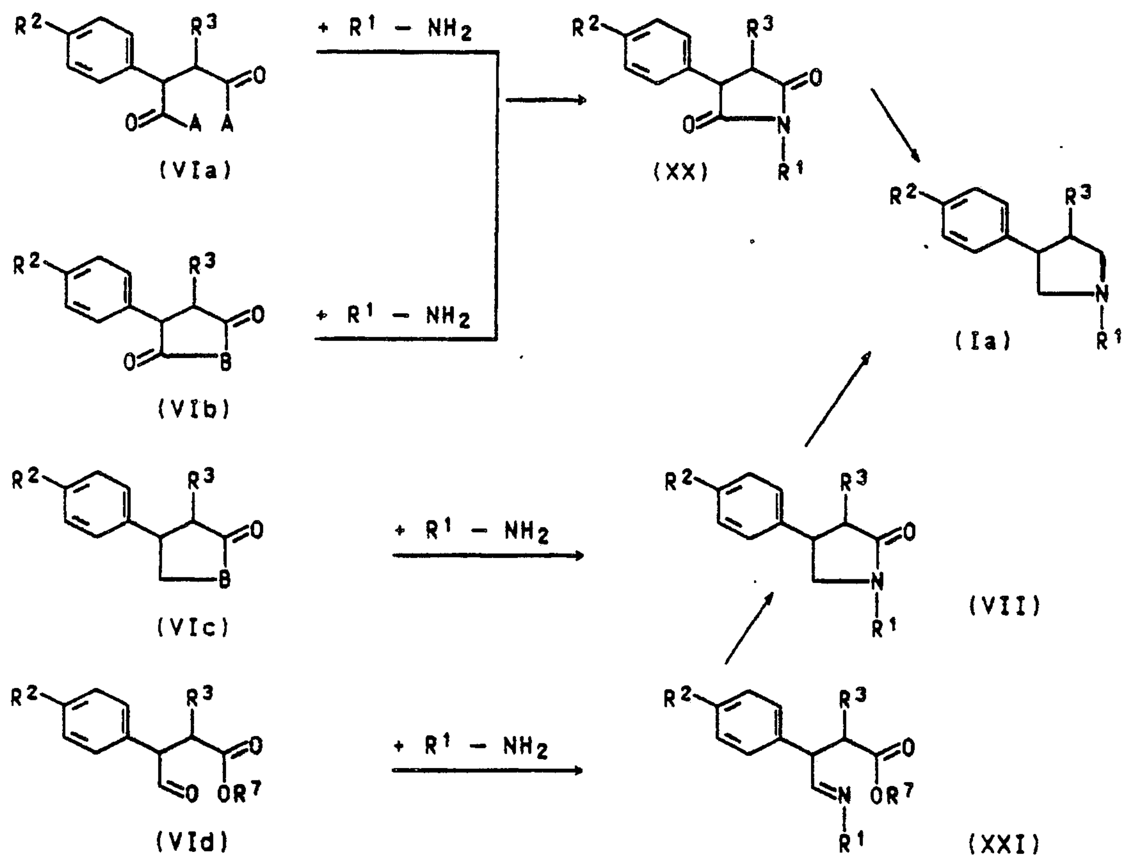


A = Hydroxy, Alkoxy oder Halogen
B = Sauerstoff oder NH
$R^7$ = Alkyl

bei der Wasserabscheidung eignen sich höher siedende Kohlenwas-serstoffe wie Toluol Xylol, Chlorbenzol oder Ligroin. Als wasserbindende Mittel eignen sich Acetanhydrid oder Molekularsiebe.

Es kann hierbei aber auch ohne Lösungsmittel gearbeitet werden. Die Reaktionen lassen sich bei Temperaturen ab 100° C durchführen. Falls die Reaktionstemperatur unter Normaldruck nicht erreichbar ist, wird im Autoklaven unter dem Eigendruck der Reaktionsmischung bei der benötigten Umsatztemperatur gearbeitet.

Die Dicarbonsäurendihalogenide lassen sich bei -20 bis +100° C, vorzugsweise bei 0 bis +10° C in Gegenwart einer Base, die zusätzlich als Lösungsmittel dienen kann, wie beispielsweise Diisopropylamin, Tributylamin, Pyridin, Picolin, Triethylamin, Dicycloheylamin oder einer Base wie beispielsweise Natriumcarbo- nat, Kaliumcarbonat oder Natriumhydrogencarbonat in Gegenwart eines inerten Lösungsmittels wie Chloroform, Dichlormethan oder Tetrahydrofuran umsetzen.

Die Umsetzung der Aminderivate $R^1$-$NH_2$ mit einem 5-Ring-Lacton-Derivat (VIc, B=NH) findet bei hohen Temperaturen, die zwischen 150 und 280° C liegen, leichter statt, wobei die benötigte Reaktionstemperatur gegebenenfalls nur in einem Autoklaven erzielt werden kann. Die Reaktion kann in einem inerten Lösungsmittel wie beispielsweise Toluol, Xylol, Ethanol, Isopropanol oder Cyclohexanol erfolgen oder auch in Abwesenheit eines Lösungsmittels.

Zur Reduktion der Carbonylverbindung zur Alkylverbindung kann man mit einem Reduktionsmittel bei -20 bis +100° C, vorzugsweise 0 bis +60° C umsetzen. Als Reduktionsmittel finden bevorzugt Hydride wie Lithiumaluminiumhydrid und Diboran Verwendung.

Die Reduktion kann auch unter Wolff-Kishner-Reaktionsbedingungen oder elektrochemisch erfolgen.

Geeignete Lösungsmittel für die Hydridreaktion sind Ether wie Diethylether, Diisopropylether, Methyl-tert.-Butylether, Tetrahydrofuran und Dioxan, Kohlenwasserstoffe, wie Xylol, Toluol.

Die 3-Formyl-propionester-Derivate der allgemeinen Struktur VId lassen sich bei 0-100 °C, gegebenenfalls in Gegenwart einer wasserentziehenden Mittels wie Natriumsulfat, Magnesiumsulfat oder Molekularsieb, oder aber auch am Wasserabscheider mit einem geeigneten Schleppmittel mit den Aminen $R^1$-$NH_2$ zu den Schiff- schen Basen der allgemeinen Struktur X umsetzen.

Als Lösungsmittel eignen sich chlorierte Kohlenwasserstoffe, wie Dichlormethan, Ether, wie Tetrahydrofuran und Kohlenwasserstoffe, wie Toluol oder Xylol.

Die Verbindungen XXI lassen sich mit Reduktionsmitteln wie Natriumborhydrid oder Lithiumaluminiumhydrid oder aber auch an Katalysatoren wie Palladium-Aktivkohle oder Raney-Nickel mit Wasserstoff in die Pyrrolidon-Derivate VII überführen. Diese lassen sich, wie oben beschrieben, mit Reduktionsmitteln, wie beispielsweise Lithiumaluminiumhydrid in Ether, Tetrahydrofuran oder Dioxan, zu den erfindungsgemäßen Verbindungen Ia umsetzen.

Die quartären Salze lassen sich aus der Verbindung I durch Umsetzung mit Verbindungen der Formel $R^4$-X, worin $R^4$ und X die oben genannten Bedeutungen haben, herstellen.

Die Herstellung von am Stickstoff-Atom unsubstituierten Pyrrolidinen kann wie folgt durchgeführt werden.

a) Synthese von 3-Alkyl-4-phenyl-pyrrolidin auf Basis von entsprechend substituierten Zimtalkoholen

1. Ein Zimtalkohol der Formel XXII wird um eine Hydroformylierung gut durchführen zu können, mit Essigsäureanhydrid verestert (Selektivität >97 %).

2. Der "Zimtalkoholester" VIII wird mit Hilfe von Rhodium-Komplex-Katalysatoren mit CO und Wasserstoff umgesetzt. Die Formylgruppe tritt überwiegend in die α-Position zum Aromaten ein. Das Hydroformylierungsprodukt wird durch Fraktionisierung gereinigt (Selektivität >90 %).

3. Der Aldehydester IX wird mit Hilfe von Kobalt- bzw. Nickel-Katalysatoren mit Ammoniak und Wasserstoff umgesetzt. Es tritt zunächst Umsetzung an der Carbonylgruppe ein; in der Folge wandert die Acetylgruppe zur Aminogruppe. Isoliert wird überwiegend das Acetylpyrrolidinderivat XI.
Ein Anteil wird als "4-Amino-butanol-derivat" X erhalten.

4. Um den Ringschluß beim 4-Aminoalkohol X zu vervollständigen und gleichzeitig die Acetylgruppe zu verseifen, wird mit Schwefelsäure bei 50 °C behandelt.

5. Nach Alkalischstellen kann das 3-Alkyl-4-Aryl-pyrrolidin II mit Toluol extrahiert werden. Die Reinigung erfolgt duroh Fraktionieren unter vermindertem Druck (Selektivität der Stufen 3-5 ca. 60 %).

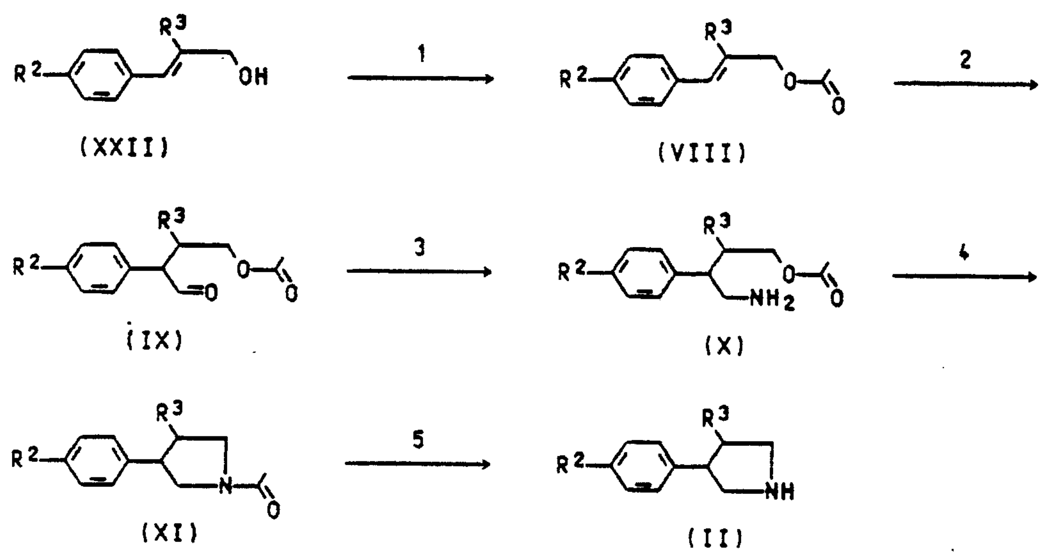


(XXII) (VIII) (IX) (X) (XI) (II)

b) Synthese von 3-Alkyl-4-aryl-pyrrolidin auf Basis von entsprechend substituierten Zimtaldehyden

1. Die Zimaldehyde XII lassen sich als ungesättigte Carbonylverbindungen in dieser Form nicht hydroformylieren. Um diese Aldehyde umsetzen zu können, ist ein Aufheben der Konjugation erforderlich. Dies läßt sich durch ein Überführen in ein Acetal erreichen. Die Acetalisierung wird zweckmäßig mit Glykol als Acetalisierungskomponente mit Hilfe einer mäßig starken Säure durchgeführt.
(Selektivität bei Rückführung des nicht umgesetzten Zimtaldehyds >85 %).

2. Die Hydroformylierung des Acetals XXIII kann mit Hilfe von Rhodium-Olefin-Komplex-Katalysatoren bei 100 °C und 650 mbar/CO:$H_2$ = 1:1 erreicht werden. Die Reinigung des Succindialdehydhalbacetals XIII kann durch Fraktionierung erfolgen.
(Selektivität >80 %).

3. Das 4-Amino-butanalacetal XIV wird durch aminierende Hydrierung mit Hilfe von Co- bzw. Ni-Katalyse synthetisiert. Das Aminoacetal XIV läßt sich gleichfalls durch fraktionierte Destillation unter vermindertem Druck reinigen. (Selektivität >80 %).

4. Der Ringschluß zu XXIV unter gleichzeitiger Reduktion wird durch Kochen mit Ameisensäure erzielt. Die Selektivität hängt sehr stark von den Substituenten am Aromaten ab.

5. Die Verseifung des Amids läßt sich zweckmäßig durch Verkochen mit Schwefelsäure, Freisetzen der Base durch Alkalischstellen und Extraktion mit Toluol erreichen. Die Reinigung des 3-Alkyl-4-aryl-pyrrolidins II wird durch fraktionierte Destillation erzielt.

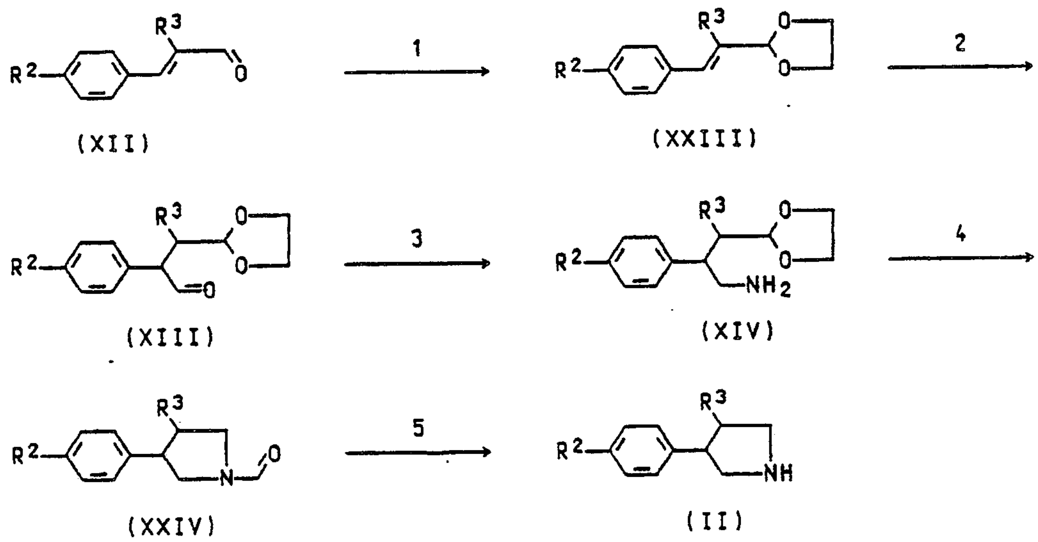


(XII) (XXIII) (XIII) (XIV) (XXIV) (II)

c) Synthese von 3-Alkyl-4-phenyl-pyrrolidin auf Basis von entsprechend substituierten Zimtsäureestern

1. Ein entsprechend substituierter Zimtsäureester XV wird unter Rhodiumoxyd-Hydrat-Katalyse bei erhöhtem Druck und bei erhöhter Temperatur mit CO und $H_2$ umgesetzt. Dabei bildet sich fast ausschließlich der entsprechende 4-Oxo-buttersäureester (VId).
Die Reinigung erfolgt durch Fraktionierung unter vermindertem Druck.
Selektivität etwa 75 %.

2. Der Oxobuttersäureester (VId) wird durch Hydrierung in Gegenwart von Cu- oder anderen Hydrierkatalysatoren in den 4-Hydroxy-buttersäureester XVI überführt. Dieser spaltet bei der Reinigung durch Destillation Alkohol ab und geht in das entsprechende $\alpha$-Butyrolacton XVII über.
(Selektivität der Hydrierung >90 %).

3. Die Überführung des Lactons XVII in das Lactam XVIII erfolgt durch Verdrücken mit wäßrigem Ammoniak bei 250 °C unter Stickstoffdruck. Das Lactam kann durch Fraktionierung unter vermindertem Druck gereinigt werden.
(Selektivität >90 %).

4. Die Reduktion der Carbonylgruppe in XVIII wird am leichtesten mit Lithiumaluminiumhydrid erreicht.
(Selektivität >90 %).

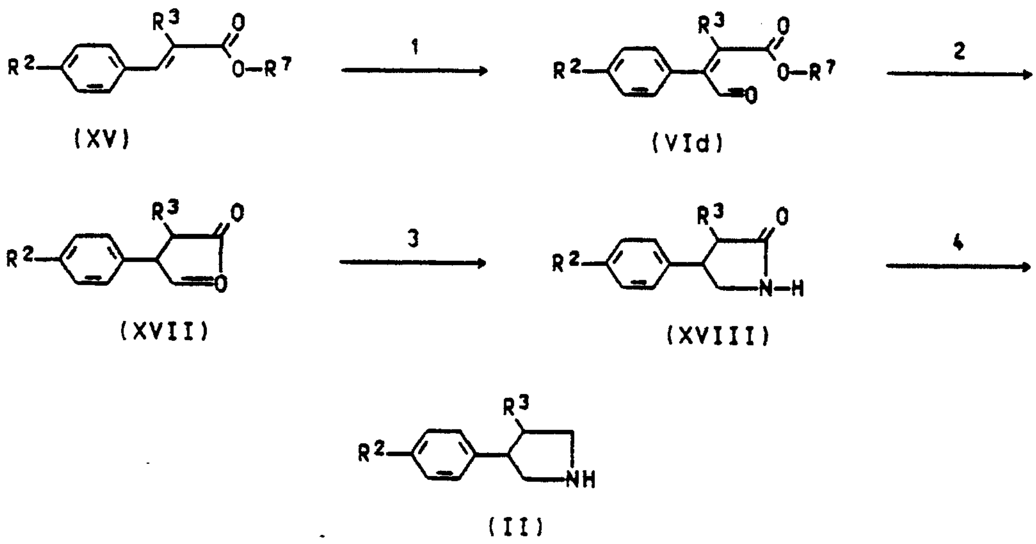


(XV) (VId) (XVII) (XVIII) (II)

Die folgenden Beispiele erläutern die Herstellung der neuen Verbindungen.

Herstellungsbeispiele

Beispiel 1

N-(4-tert.-Butyl-cyclohexyl)-3-methyl-4(4-tert.-butyl-phenyl)-pyrrolidin (Verbindung Nr. 92)

2,17 g (10 mmol) 3-Methyl-4(4-tert.-butylphenyl)-pyrrolidin (80 % trans-Isomer), 1,54 g (10 mmol) 4-tert.-Butyl-cyclohexanon, 0,75 g (5,5 mmol) Zink(II)chlorid und 0,70 g (11 mmol) Natriumcyanborhydrid werden in1 00 ml absolutem Methanol 48 h bei 20° C gerührt. Das Lösungsmittel wird i.Vak. verdampft und der Rückstand in 5 %iger Natronlauge und Methyl-tert.-butyl-ether gelöst. Die organische Phase wird mit Kochsalz-Lösung und Wasser gewaschen, getrocknet, i.Vak. verdampft und der Rückstand bei 0,4 bar bis 140° C andestilliert. Ausbeute: 1,9 g (54 %) eines zähen Harzes.

Beispiel 2

N(2,3,5,5-Tetramethyl-hexyl-3-methyl-4(p-tert.-butyl-phenyl)-pyrrolidin (Verbindung Nr. 46)

72 g 3-Methyl-4-(p-tert.butyl-phenyl)-pyrrolidin werden mit 57 g Tetra(2,3,5,5)-methyl-hexanal versetzt und mit Hilfe von Xylol im Verlauf von 6,5 Stunden am Rückfluß gekocht und entstehendes Wasser abgetrennt. Es werden 4,2 g Wasser abgetrennt. Zu der Reaktionslösung werden 16 g Ameisensäure zugetropft und bei 132 bis 142° C unter Rühren zum Sieden erhitzt. Die $CO_2$-Abspaltung ist nach 4 Stunden beendet. Das Reaktionsprodukt wird durch Kurzwegfraktionierung bei einem Druck von 2 mbar gereinigt. Als Vorlauf fallen bis zu einer Übergangstemperatur von 168° C 9,9 g an. Die Hauptmenge geht zwischen 168 bis 170° C über = 93 g. Destillationsrückstand = 4 g. Nach H-NMR- und IR_Spektrum liegt das 1-(2,3,5,5-Tetramethyl-hexyl)-3-methyl-4-(p-tert.butyl-phenyl)-pyrrolidin als Isomerengemisch vor.

Beispiel 3

Herstellung des 1-Methyl-1-(2,3,5,5-tetramethyl-hexyl)-3-methyl-4-(p-tert.butyl-phenyl)-pyrrolidinium-bromids (Verbindung Nr. 189)

40 g (1-2,3,5,5-Tetramethyl-hexyl)-3-methyl-4-(p-tert.butyl-phenyl)-pyrrolidin werden mit 30 g Tetrahydrofuran versetzt. Zur Bildung des quartären Salzes werden 40 g einer Lösung von Methylbromid in Acetonitril (20 %ig) zugesetzt. Beim Stehen über Nacht kristallisiert das quartäre Salz aus. Beim Absaugen werden 20,5 g isoliert. Fp. liegt bei 250 °C. Bei 270 °C tritt starke Verfärbung auf.

Die folgenden Verbindungen können in entsprechender Weise hergestellt werden.

Tabelle 1

n=0

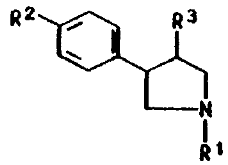

I

| Verb. Nummer | $R^1$ | $R^2$ | $R^3$ | | IR-Absorption ($cm^{-1}$) [Film] |
|---|---|---|---|---|---|
| 1 | n-Propyl | tert.-Butyl | Methyl | | |
| 2 | Isopropyl | tert.-Butyl | Methyl | | |
| 3 | n-Butyl | tert.-Butyl | Methyl | | |
| 4 | Isobutyl | tert.-Butyl | Methyl | | |
| 3 | sec.-Butyl | tert.-Butyl | Methyl | | |
| 4 | tert.-Butyl | tert.-Butyl | Methyl | | |
| 5 | n-Pentyl | tert.-Butyl | Methyl | | |
| 6 | 3-Methyl-butyl | tert.-Butyl | Methyl | | |
| 7 | 2,2-Dimethyl-propyl | tert.-Butyl | Methyl | Öl | 2954,2904,2868,2779,1510,1478,1463,1394,1361,1129,829,573. |
| 8 | Pent-2-yl | tert.-Butyl | Methyl | | |
| 9 | Pent-3-yl | tert.-Butyl | Methyl | | |
| 10 | 1,2-Dimethyl-propyl | tert.-Butyl | Methyl | | |
| 11 | n-Hexyl | tert.-Butyl | Methyl | | |
| 12 | Hex-2-yl | tert.-Butyl | Methyl | | |
| 13 | Hex-3-yl | tert.-Butyl | Methyl | | |
| 14 | 1,2,2-Trimethyl-propyl | tert.-Butyl | Methyl | | |
| 15 | 1,3-Dimethyl-butyl | tert.-Butyl | Methyl | | |
| 16 | 4-Methyl-pentyl | tert.-Butyl | Methyl | | |
| 17 | 3,3-Dimethyl-butyl | tert.-Butyl | Methyl | Öl | 2955,2909,2868,2800,1510,1476,1465,1363,829,574. |
| 18 | n-Heptyl | tert.-Butyl | Methyl | | |

Tabelle 1 (Fortsetzung)

| Verb. nummer | $R^1$ | $R^2$ | $R^3$ | IR-Absorption ($cm^{-1}$) [Film] |
|---|---|---|---|---|
| 19 | Hept-2-yl | tert.-Butyl | Methyl | |
| 20 | Hept-3-yl | tert.-Butyl | Methyl | |
| 21 | Hept-4-yl | tert.-Butyl | Methyl | |
| 22 | Diisopropyl-methyl | tert.-Butyl | Methyl | |
| 23 | 1,4-Dimethyl-pentyl | tert.-Butyl | Methyl | |
| 24 | 4,4-Dimethyl-pentyl | tert.-Butyl | Methyl | Sdp. 166-170°C/2 mbar |
| 25 | n-Octyl | tert.-Butyl | Methyl | |
| 26 | 2-Methyl-hept-3-yl | tert.-Butyl | Methyl | |
| 27 | 5-Methyl-hept-3-yl | tert.-Butyl | Methyl | |
| 28 | Oct-2-yl | tert.-Butyl | Methyl | |
| 29 | Oct-3-yl | tert.-Butyl | Methyl | |
| 30 | Oct-4-yl | tert.-Butyl | Methyl | |
| 31 | 5,5-Dimethyl-hexyl | tert.-Butyl | Methyl | |
| 32 | 2,4,4-Trimethyl-pentyl | tert.-Butyl | Methyl | Öl 2955,2910,2868,1476,1462, 1375,1363,829,573. |
| 33 | 6-Methyl-hept-2-yl | tert.-Butyl | Methyl | Sdp. 145°C/0,4 mbar |
| 34 | n-Nonyl | tert.-Butyl | Methyl | |
| 35 | Non-2-yl | tert.-Butyl | Methyl | |
| 36 | Non-3-yl | tert.-Butyl | Methyl | |
| 37 | Non-4-yl | tert.-Butyl | Methyl | |
| 38 | Non-5-yl | tert.-Butyl | Methyl | |
| 39 | 2,5,5-Trimethyl-hexyl | tert.-Butyl | Methyl | |
| 40 | 2,6-Dimethyl-hept-4-yl | tert.-Butyl | Methyl | |
| 41 | 3,5,5-Trimethyl-hexyl | tert.-Butyl | Methyl | Sdp. 170-175°C/2 mbar |
| 42 | n-Decyl | tert.-Butyl | Methyl | |
| 43 | Dec-2-yl | tert.-Butyl | Methyl | |
| 44 | Dec-3-yl | tert.-Butyl | Methyl | |
| 45 | Dec-4-yl | tert.-Butyl | Methyl | |

Tabelle 1 (Fortsetzung)

| Verb. Nummer | $R^1$ | $R^2$ | $R^3$ | IR-Absorption ($cm^{-1}$) [Film] |
|---|---|---|---|---|
| 46 | 2,3,5,5-Tetramethyl-hexyl | tert.-Butyl | Methyl | |
| 47 | n-Undecyl | tert.-Butyl | Methyl | |
| 48 | n-Dodecyl | tert.-Butyl | Methyl | |
| 49 | n-Tridecyl | tert.-Butyl | Methyl | |
| 50 | 1,5,9-Trimethyl-decyl | tert.-Butyl | Methyl | |
| 51 | n-Tetradecyl | tert.-Butyl | Methyl | |
| 52 | 2-Hydroxyethyl | tert.-Butyl | Methyl | |
| 53 | 3-Hydroxypropyl | tert.-Butyl | Methyl | |
| 54 | 4-Hydroxy-butyl | tert.-Butyl | Methyl | |
| 55 | 1-hydroxy-but-2-yl | tert.-Butyl | Methyl | |
| 57 | 2-Hydroxy-but-3-yl | tert.-Butyl | Methyl | |
| 58 | 3-Chlorpropyl | tert.-Butyl | Methyl | |
| 59 | 6-Chlorhexyl | tert.-Butyl | Methyl | |
| 60 | Trifluorethyl | tert.-Butyl | Methyl | |
| 61 | Trichlorethyl | tert.-Butyl | Methyl | |
| 62 | 5-Chlor-pent-2-yl | tert.-Butyl | Methyl | |
| 63 | 3-Chlor-but-2-yl | tert.-Butyl | Methyl | |
| 64 | 3,3-Dichlor-prop-2-yl | tert.-Butyl | Methyl | |
| 65 | 2-Methoxyethyl | tert.-Butyl | Methyl | |
| 66 | 2-Ethoxyethyl | tert.-Butyl | Methyl | |
| 67 | 2-tert.-Butoxyethyl | tert.-Butyl | Methyl | |
| 68 | 3-tert.-Butoxy-ethyl | tert.-Butyl | Methyl | |
| 69 | 6-Methoxyhexyl | tert.-Butyl | Methyl | |
| 70 | 3-Methoxy prop 2-yl | tert.-Butyl | Methyl | |
| 71 | Trimethylsilylethyl | tert.-Butyl | Methyl | |
| 72 | 3-Trimethylsilyl-propyl | tert.-Butyl | Methyl | |
| 73 | 6-Trimethylsilyl hexyl | tert.-Butyl | Methyl | |
| 74 | Allyl | tert.-Butyl | Methyl | |

Tabelle 1 (Fortsetzung)

| Verb. Nummer | $R^1$ | $R^2$ | $R^3$ | | IR-Absorption ($cm^{-1}$) [Film] |
|---|---|---|---|---|---|
| 75 | 3-Methyl-but-2-en-yl | tert.-Butyl | Methyl | | |
| 76 | 1,5,9-Trimethyl-deca-4,8-chenyl | tert.-Butyl | Metyhl | | |
| 77 | Propargyl | tert.-Butyl | Methyl | | |
| 78 | 4,4-Dimethyl-but-in-1-yl | tert.-Butyl | Methyl | | |
| 79 | Cyclopentyl | tert.-Butyl | Methyl | | |
| 80 | Cyclohexyl | tert.-Butyl | Methyl | Öl | 2957,2928,2854,2775,1509, 1462,1450,1374,1363,829. |
| 81 | Cycloheptyl | tert.-Butyl | Methyl | | |
| 82 | 4-Hydroxy-cyclohexyl | tert.-Butyl | Methyl | | |
| 83 | 4-tert.-Butoxy-cyclohexyl | tert.-Butyl | Methyl | | |
| 84 | 4-Trimethylsolyl-cyclohexyl (cis-trans-Gemisch) | tert.-Butyl | Methyl | | |
| 85 | Cyclohex-2-en-yl | tert.-Butyl | Methyl | | |
| 86 | 4-Methyl-cyclohexyl | tert.-Butyl | Methyl | | |
| 87 | 3-Methyl-cyclohexyl | tert.-Butyl | Methyl | | |
| 88 | 3,3-Dimethyl-cyclohexyl | tert.-Butyl | Methyl | Öl | 2952,2925,2865,2781,1509, 1475,1461,1385,1363,1122, 829,574. |
| 89 | 3,3,5-Trimethylcyclohexyl | tert.-Butyl | Methyl | | |
| 90 | 4-Isopropyl-cyclohexyl (Isomerengemisch) | tert.-Butyl | Methyl | Öl | 2956,2934,2867,2774,1509, 1475,1462,1450,1375,1366, 828,574. |
| 91 | trans-4-Isopropyl-cyclohexyl | tert.-Butyl | Methyl | | |
| 92 | 4-tert.-Butyl-cyclohexyl (Isomerengemisch) | tert.-Butyl | Methyl | | |
| 93 | trans-4-tert.Butyl-cyclohexyl | tert.-Butyl | Methyl | | |
| 94 | 4(2-Methyl-but-2-yl)cyclohexyl (Isomerengemisch) | tert.-Butyl | Methyl | | |

Tabelle 1 (Fortsetzung)

| Verb. Nummer | $R^1$ | $R^2$ | $R^3$ | | IR-Absorption ($cm^{-1}$) [Film] |
|---|---|---|---|---|---|
| 95 | trans-4(2-Methyl-but-2-yl)-cyclohexyl | tert.-Butyl | Methyl | | |
| 96 | 4(2,4,4-Trimethyl-pent-2-yl) cyclohexy (Isomerengemisch) | tert.-Butyl | Methyl | | |
| 97 | trans-4(2,4,4-Trimethyl-pent-2-yl)cyclohexyl | tert.-Butyl | Methyl | | |
| 98 | 4-Hydroxy-3-methyl-cyclohexyl | tert.-Butyl | Methyl | | |
| 99 | 4-Hydroxy-3,6-dimethyl-cyclohexyl | tert.-Butyl | Methyl | | |
| 100 | 4-Hydroxy-3,3-dimethyl-cyclohexyl | tert.-Butyl | Methyl | | |
| 101 | 4-Hydroxy-3,3,5-trimethyl-cyclohexyl | tert.-Butyl | Methyl | Harz | 3440,2958,2928,2868,1475,1460,1376,1363,1333,829,575. |
| 102 | Cyclohexylmethyl | tert.-Butyl | Methyl | | |
| 103 | Cyclohexylethyl | tert.-Butyl | Methyl | | |
| 104 | 4-tert.-Buyl-cyclohex-3-en-yl | tert.-Butyl | Methyl | | |
| 105 | 4-tert.-Butyl-cyclohex-2-en-yl | tert.-Butyl | Methyl | | |
| 106 | 1-Decalyl (cis/trans-Gemisch) | tert.-Butyl | Methyl | | |
| 107 | 2-Decalyl (cis/trans-Gemisch) | tert.-Butyl | Methyl | Harz | 2956,2921,2862,2776,1509,1476,1459,1448,1375,1362,829,575. |
| 108 | trans-2-Decalyl (eq/ax.-substituiert) | tert.-Butyl | Methyl | | |
| 109 | eq.-trans-2-Decalyl | tert.-Butyl | Methyl | | |
| 110 | 6-Hydroxy-2-decalyl | tert.-Butyl | Metyhl | | |
| 111 | 7-Hydroxy-2-decalyl | tert.-Butyl | Methyl | | |
| 112 | 2-Decalylmethyl | tert.-Butyl | Methyl | | |
| 113 | 9-Methyl-trans-2-decalyl | tert.-Butyl | Methyl | | |
| 114 | 5,9-Dimethyl-trans-2-decalyl | tert.-Butyl | Methyl | | |

Tabelle 1 (Fortsetzung)

| Verb. Nummer | $R^1$ | $R^2$ | $R^3$ | IR-Absorption ($cm^{-1}$) [Film] |
|---|---|---|---|---|
| 115 | 5,5,9-Trimethyl-trans-2-decalyl | tert.-Butyl | Methyl | |
| 116 | 6-Hydroxy-9-methyl-2-decalyl | tert.-Butyl | Methyl | |
| 117 | 6-Hydroxy-5,9-dimethyl-2-decalyl | tert.-Butyl | Methyl | |
| 118 | 6-Hydroxy-5,5,9-trimethyl2-decalyl | tert.-Butyl | Methyl | |
| 119 | Tetrahydropyran-4-yl | tert.-Butyl | Methyl | Öl 2956,2924,2867,2773,1362,1174,1164,1095,831. |
| 120 | Tetrahydrothiopyran-4-yl | tert.-Butyl | Methyl | |
| 121 | 1,4-Dioxan-2-yl-methyl | tert.-Butyl | Methyl | Sdp. 190-200°C/2 mbar |
| 122 | Tetrahydropyran-2-yl-methyl | tert.-Butyl | Methyl | Sdp. 160-175°C/2 mbar |
| 123 | Tetrahydropyran-3-yl-methyl | tert.-Butyl | Methyl | Sdp. 156-158°C/2 mbar |
| 124 | 3,5-Dimethyl-dioxan-2-yl-methyl | tert.-Butyl | Methyl | Sdp. 177-190°C/3 mbar |
| 125 | 3,5-Diethyl-dioxan-2-yl-methyl | tert.-Butyl | Methyl | Sdp. 168-172°C/3 mbar |
| 126 | 3,6-Diethyl-dioxan-2-yl-methyl | tert.-Butyl | Methyl | Sdp. 168-172°C/3 mbar |
| 127 | Phenyl | tert.-Butyl | Methyl | |
| 128 | 4-Hydroxy-phenyl | tert.-Butyl | Methyl | |
| 129 | 4-Chlor-phenyl | tert.-Butyl | Methyl | |
| 130 | 4-tert.-Butoxy-phenyl | tert.-Butyl | Methyl | |
| 131 | Benzyl | tert.-Butyl | Methyl | Sdp. 176-180°C/4 mbar |
| 132 | 4-Chlor-benzyl | tert.-Butyl | Methyl | |
| 133 | 2,4-Dichlorbenzyl | tert.-Butyl | Methyl | |
| 134 | 4-tert.-Butoxybenzyl | tert.-Butyl | Methyl | |
| 135 | 4-Trimethylsolylbenzyl | tert.-Butyl | Methyl | |
| 136 | 4-Phenyl-4-methyl-pentyl | tert.-Butyl | Methyl | Sdp. 220°C/4 mbar |
| 137 | 4-tert.-Butylbenzyl | tert.-Butyl | Methyl | Sdp. 210°C/4 mbar |
| 138 | 4-tert.-Butylphenylethyl | tert.-Butyl | Methyl | |
| 139 | 4-tert.-Butylphenylpropyl | tert.-Butyl | Methyl | |
| 140 | 3,3-Dimethyl-butyl | 2-Methyl-but-2-yl | Methyl | |

Tabelle 1 (Fortsetzung)

| Verb. Nummer | $R^1$ | $R^2$ | $R^3$ | IR-Absorption ($cm^{-1}$) [Film] |
|---|---|---|---|---|
| 141 | 3,5,5-Trimethyl-hexyl-cyclohexyl | 2-Methyl-but-2-yl | Methyl | |
| 142 | 3-Methyl-cyclohexyl | 2-Methyl-but-2-yl | Methyl | |
| 143 | 3,3-Dimethyl-cyclohexyl | 2-Methyl-but-2-yl | Methyl | |
| 144 | 4-Isopropyl-cyclohexyl | 2-Methyl-but-2-yl | Methyl | |
| 145 | 4-tert.-Butyl-cyclohexyl (Isomerengemisch) | 2-Methyl-but-2-yl | Methyl | |
| 146 | trans-4-tert.-Butyl-cyclohexyl | 2-Methyl-but-2-yl | Methyl | |
| 147 | 4-tert.-Butyl-cyclohexyl | 2-Methyl-but-2-yl | Methyl | |
| 148 | 4-Trimethylsilylcyclohexyl | 2-Methyl-but-2-yl | Methyl | |
| 149 | 4-Hydroxy-cyclohexyl | 2-Methyl-but-2-yl | Methyl | |
| 150 | 3,3,5-Trimethyl-cyclohexyl | 2-Methyl-but-2-yl | Methyl | |
| 151 | 2-Decalyl | 2-Methyl-but-2-yl | Methyl | |
| 152 | 6-Hydroxy-2-decalyl | 2-Methyl-but-2-yl | Methyl | |
| 153 | 5,5,9-Trimethyl-2-decalyl | 2-Methyl-but-2-yl | Methyl | |
| 154 | 6-Hydroxy-5,9-dimethyl-2-decalyl | 2-Methyl-but-2-yl | Metyhl | |
| 155 | 6-Hydroxy-5,5,9-Trimethyl-2-decalyl | 2-Methyl-but-2-yl | Methyl | |
| 156 | 4-Isopropyl-cyclohexyl | 2,4,4-Trimethyl-pent-2-yl | Methyl | |
| 157 | 3,3-dimethyl-cyclohexyl | 2,4,4-Trimethyl-pent-2-yl | Methyl | |
| 158 | 3,3-Dimethyl-butyl | 2,4,4-Trimethyl-pent-2-yl | Methyl | |
| 159 | 4-tert.-Butyl-cyclohexyl | 2,4,4-Trimethyl-pent-2-yl | Methyl | |
| 160 | 4-Trimethylsilyl-cyclohexyl | 2,4,4-Trimethyl-pent-2-yl | Methyl | |
| 161 | 2 Decalyl | 2,4,4-Trimethyl-pent-2-yl | Methyl | |
| 162 | 6-Hydroxy-2-decalyl | 2,4,4-Trimethyl-pent-2-yl | Methyl | |
| 163 | 5,5,9-Trimethyl-2-decalyl | 2,4,4-Trimethyl-pent-2-yl | Methyl | |
| 164 | 6-Hydroxy-5,9-dimethyl-decalyl | 2,4,4-Trimethyl-pent-2-yl | Methyl | |
| 165 | 4 tert.-Butyl-cyclohexyl | tert.-Butoxy | Methyl | |
| 166 | 4-Trimethylsilyl-cylclohexyl | tert.-Butoxy | Methyl | |
| 167 | 3,3-Dimethyl-dutyl | tert.-Butoxy | Methyl | |

Tabelle 1 (Fortsetzung)

| Verb. Nummer | $R^1$ | $R^2$ | $R^3$ | IR-Absorption ($cm^{-1}$) [Film] |
|---|---|---|---|---|
| 168 | 3,3-Dimethyl-cyclohexyl | tert.-Butoxy | Methyl | |
| 169 | 4-Hydroxy-3,3,5-trimethyl-cyclohexyl | tert.-Butoxy | Methyl | |
| 170 | 4-tert.-Butyl-cyclohexyl | tert.-Butyl | Ethyl | |
| 171 | 4-tert.-Butyl-cyclohexyl | tert.-Butyl | Propyl | |
| 172 | 4-tert.-Butyl-cyclohexyl | tert.-Butyl | Butyl | |
| 173 | 3(4-tert.-Butyl-phenyl)2-methyl-propyl | tert.-Butyl | Methyl | Sdp. 215-220°C/2 mbar |
| 174 | 3,6-Dimethyl-dioxan-2-yl-methyl | tert.-Butyl | Methyl | Sdp. 177-190°C/3 mbar |

eq/ax = Substitution in äquatorialer oder axialer Position

Tabelle 2

n=1

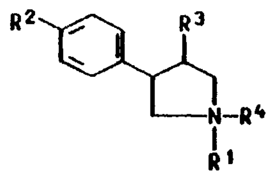

X⁻    I

| Verbindungs-Nummer | $R^1$ | $R^2$ | $R^3$ | $R^4$ | $X^-$ | |
|---|---|---|---|---|---|---|
| 175 | 4-tert.-Butyl-cyclohexyl | tert.-Butyl | Methyl | Methyl | J | |
| 176 | 4-tert.-Butyl-cyclohexyl | tert.-Butyl | Methyl | Methyl | Cl | |
| 177 | 4-tert.-Butyl-cyclohexyl | tert.-Butyl | Methyl | Ethyl | J | |
| 178 | 2-Decalyl | tert.-Butyl | Methyl | Methyl | J | |
| 179 | 2-Decalyl | tert.-Butyl | Methyl | Methyl | Cl | |
| 180 | 6-Hydroxy-2-decalyl | tert.-Butyl | Methyl | Methyl | J | |
| 181 | 5,5,9-Trimethyl-2-decalyl | tert.-Butyl | Methyl | Methyl | J | |
| 182 | 5,5,9-Trimethyl-2-decalyl | tert.-Butyl | Methyl | Methyl | Cl | |
| 183 | 6-Hydroxy-5,5,9-Trimethyl-2-decalyl | tert.-Butyl | Methyl | Methyl | Cl | |
| 184 | 3,3,5-Trimethyl-hexyl | tert.-Butyl | Methyl | Methyl | J | Fp. 145-175°C |
| 185 | 3(4-tert.-Butylphenyl)-2-methyl-propyl | tert.-Butyl | Methyl | Methyl | J | Fp. 168-185°C |
| 186 | Tetrahydropyran-3-yl-methyl | tert.-Butyl | Methyl | Methyl | J | Fp. 180-188°C |
| 187 | 4-tert.-Butyl-benzyl | tert.-Butyl | Methyl | Alkyl | Br | Fp. 105-114°C |
| 188 | 2,3,5,5-Tetramethyl-hexyl | tert.-Butyl | Methyl | Methyl | J | Fp. 219-245°C |
| 189 | 2,3,5,5-Tetramethyl-hexyl | tert.-Butyl | Methyl | Methyl | Br | Fp. 250-270°C |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen.
Plasmopara viticola an Reben,
Alternaria-Arten an Obst und Gemüse.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Ver teilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Einige der neuen Verbindungen haben sehr gute Wirksamkeit gegen humanpathogene Pilze, wie Trichophyton mentagrophytes und Candida albicans.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 41 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 121 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 24 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 122 werden in einer Mischung gelöst, die aus 25 Gew.-Teilen Cyclohexanol, 65 Gew.-Teilen einer Mineralölfraktion vom Siedepunkt 210 bis 280° C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 123 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 124 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 131 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 136 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 137 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,

N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoff wurde N-(2-Methyl-3-(p-tert.butylphenyl)-propylpyrrolidin (A) - bekannt aus DE-2 727 482 - benutzt.

Anwendungsbeispiel 1

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten kammer aufgestellt. Dann erfolgte die Beurteilung des Ausmaßes des Pilzausbruches auf den Blattunterseiten.

Das Ergebnis zeigt, daß die Wirkstoffe 24, 41, 121, 122, 123, 124, 131, 137, 136, 173, 189, 184, 185 und 188 bei der Anwendung als 0,05 %ige (Gew.%) Spritzbrühe eine bessere fungizide Wirkung (97 %) zeigen als der bekannte Vergleichswirkstoff A (60 %).

Anwendungsbeispiel 2

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Onidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 41, 121, 122, 123, 124, 137, 136, 173, 184, 185, 186, 187 und 188 bei der Anwendung als 0,05 %ige Spritzbrühe eine bessere fungizide Wirkung (90 %) zeigten als der bekannte Vergleichswirkstoff A (50 %).

Anwendungsbeispiel 3

Wirksamkeit gegen Gerstenmehltau

Blätter von in Töpfen gewachsenen Gerstenkeimlingen der Sorte "Igri" wurden mit wäßriger Spritzbrühe, die 80 % Wirkstoff und 20 % Emulgiermittel in der Trockensubstanz enthielt, besprüht und nach 24 Stunden mit Oidien (Sporen) des Gerstenmehltaus (Erysiphe graminis f. sp. hordei) bestäubt. Die Versuchspflanzen wurden anschließend im Gewächshaus bei Temperaturen zwischen 20 und 22° C und 75 b is 80 % relativer Luftfeuchtigkeit aufgestellt. Nach 7 Tagen wurde das Ausmaß der Mehltauentwicklung ermittelt.

Das Ergebnis zeigt, daß die Wirkstoffe 24, 41, 121, 122, 123, 124, 131, 185 und 188 bei der Anwendung als 0,006 und 0,0015 %ige Spritzbrühe eine bessere fungizide Wirkung zeigen (97 %) als der bekannte Vergleichswirkstoff A (70 %).

## Ansprüche

1. N-substituierte 3-Alkyl-4-aryl-pyrrolidin-Derivate der Formel

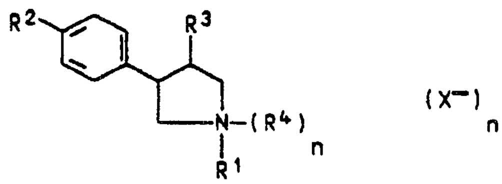

I

in der

$R^1$ gegebenenfalls durch Hydroxy, Halogen, $C_1$-$C_5$-Alkoxy oder $C_3$-$C_9$-Trialkylsilyl substituiertes $C_2$-$C_{20}$-Alkyl, $C_3$-$C_{20}$-Alkenyl, $C_3$-$C_{20}$-Alkinyl, gegebenenfalls substituiertes $C_4$-$C_{12}$-Cycloalkyl, $C_4$-$C_{12}$-Cycloalkenyl, gegebenenfalls substituiertes $C_4$-$C_{20}$-Alkyl-cycloalkyl, $C_4$-$C_{20}$-Cycloalkyl-alkyl, $C_4$-$C_{20}$-Alkyl-cycloalkyl-alkyl, $C_4$-$C_{20}$-Cycloalkenyl-alkyl, $C_4$-$C_{20}$-Alkyl-cycloalkenyl, gegebenenfalls substituiertes $C_9$-$C_{11}$-Bicycloalkyl, $C_{10}$-$C_{15}$-Bicycloalkylalkyl, gegebenenfalls substituiertes $C_{10}$-$C_{15}$-Alkyl-bicycloalkyl,
5 bis 7-gliedriges Heterocycloalkyl mit 1 oder 2. Heteroatomen aus der Gruppe Sauerstoff und/oder Schwefel, 5 bis 7-gliedriges Heterocycloalkylmethyl mit 1 oder 2 Heteroatomen aus der Gruppe Sauerstoff und/oder Schwefel, $C_1$-$C_8$-Alkyl-substituiertes 5 bis 7-gliedriges Heterocycloalkyl oder Heterocycloalkylmethyl mit 1 oder 2 Heteroatomen aus der Gruppe Sauerstoff und/oder Schwefel,
gegebenenfalls substituiertes Aryl, gegebenenfalls substituiertes $C_7$-$C_{20}$-Arylalkyl, $C_7$-$C_{20}$-Alkylaryl, $C_8$-$C_{20}$-Alkyl-aryl-alkyl,
$R^2$ $C_3$-$C_{10}$-Alkyl, $C_3$-$C_8$-Alkoxy,
$R^3$ $C_1$-$C_4$-Alkyl
$R^4$ $C_1$-$C_5$-Alkyl, $C_3$-$C_5$-Alkenyl, $C_3$-$C_5$-Alkinyl und $C_7$-$C_8$-Phenylalkyl,
$X^-$ ein für Pflanzen verträgliches Anion bedeutet,
n den Wert 0 oder 1 hat
und deren für Pflanzen verträgliche Salze.

2. Verfahren zur Herstellung der Verbindungen der Formel I, dadurch gekennzeichnet daß man die Verbindungen II

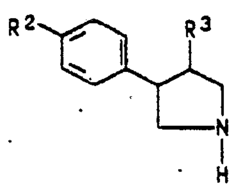

(II)

mit einer Carbonylverbindung der Formel III

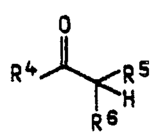

(III)

wobei die Reste $R^4$ bis $R^6$ so definiert sind, daß der Rest

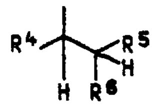

in seiner Gesamtheit dem Reste $R^1$ entspricht, entweder direkt unter reduzierenden Bedingungen umsetzt oder zu einem Enamin umsetzt und dieses katalytisch hydriert oder mit einem Reduktionsmittel reduziert, oder
für den Fall, daß $R^2 = C_3$-$C_{10}$ Alkyl ist, ein Phenylpyrrolidin-Derivat der Formel IV

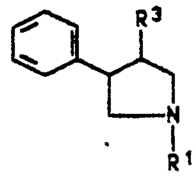

(IV)

säurekatalysiert mit einem Alken umsetzt, dessen Struktur dem Rest $R^2$ entspricht,
und gegebenenfalls die so erhaltenen Verbindungen der Formel I mit einer Säure in ihr Säureadditionssalz oder mit $R^4$-X in das quartäre Salz überführt, wobei X die obengenannte Bedeutung hat.

3. Fungizid, enthaltend eine fungizid wirksame Menge einer Verbindung der Formel I

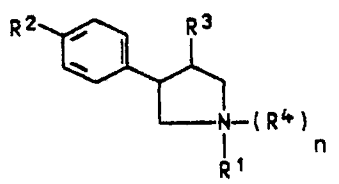

$(X^-)_n$ I

in der
$R^1$ gegebenenfalls durch Hydroxy, Halogen, $C_1$-$C_5$-Alkoxy oder $C_3$-$C_9$-Trialkylsilyl substituiertes $C_2$-$C_{20}$-Alkyl, $C_3$-$C_{20}$-Alkenyl, $C_3$-$C_{20}$-Alkinyl, gegebenenfalls substituiertes $C_4$-$C_{12}$-Cycloalkyl, $C_4$-$C_{12}$-Cycloalkenyl, gegebenenfalls substituiertes $C_4$-$C_{20}$-Alkyl-cycloalkyl, $C_4$-$C_{20}$-Cycloalkyl-alkyl, $C_4$-$C_{20}$-Alkyl-cycloalkyl-alkyl, $C_4$-$C_{20}$-Cycloalkenyl-alkyl, $C_4$-$C_{20}$-Alkyl-cycloalkenyl, gegebenenfalls substituiertes $C_9$-$C_{11}$-Bicycloalkyl, $C_{10}$-$C_{15}$-Bicycloalkylalkyl, gegebenenfalls substituiertes $C_{10}$-$C_{15}$-Alkyl-bicycloalkyl,
5 bis 7-gliedriges Heterocycloalkyl mit 1 oder 2 Heteroatomen aus der Gruppe Sauerstoff und/oder Schwefel, 5 bis 7-gliedriges Heterocycloalkylmethyl mit 1 oder 2 Heteroatomen aus der Gruppe Sauerstoff und/oder Schwefel, $C_1$-$C_8$-Alkyl-substituiertes 5 bis 7-gliedriges Heterocycloalkyl oder Heterocycloalkylmethyl mit 1 oder 2 Heteroatomen aus der Gruppe Sauerstoff und/oder Schwefel,
gegebenenfalls substituiertes Aryl, gegebenenfalls substituiertes $C_7$-$C_{20}$-Arylalkyl, $C_7$-$C_{20}$-Alkylaryl, $C_8$-$C_{20}$-Alkyl-aryl-alkyl,

$R^2$ $C_3$-$C_{10}$-Alkyl, $C_3$-$C_8$-Alkoxy,
$R^3$ $C_1$-$C_4$-Alkyl
$R^4$ $C_1$-$C_5$-Alkyl, $C_3$-$C_5$-Alkenyl, $C_3$-$C_5$-Alkinyl und $C_7$-$C_8$-Phenylalkyl,
$X^-$ ein für Pflanzen verträgliches Anion bedeutet,
n den Wert 0 oder 1 hat
oder deren für Pflanzen verträglichen Salze und einen inerten Trägerstoff.

4. Verwendung einer Verbindung der Formel I als Fungizid.

5. Verfahren zur Bekämpfung von Pilzen, dadurch gekennzeichnet, daß man die Pilze oder die vom Pilzbefall bedrohten Materialien, Pflanzen, Saatgüter oder den Boden mit einer Verbindung der Formel I

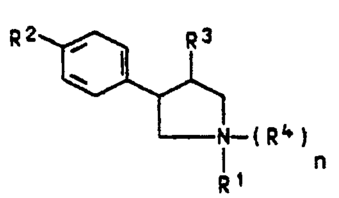

$(X^-)_n$ I

in der

$R^1$ gegebenenfalls durch Hydroxy, Halogen, $C_1$-$C_5$-Alkoxy oder $C_3$-$C_9$-Trialkylsilyl substituiertes $C_2$-$C_{20}$-Alkyl, $C_3$-$C_{20}$-Alkenyl, $C_3$-$C_{20}$-Alkinyl, gegebenenfalls substituiertes $C_4$-$C_{12}$-Cycloalkyl, $C_4$-$C_{12}$-Cycloalkenyl, gegebenenfalls substituiertes $C_4$-$C_{20}$-Alkyl-cycloalkyl, $C_4$-$C_{20}$-Cycloalkyl-alkyl, $C_4$-$C_{20}$-Alkyl-cycloalkyl-alkyl, $C_4$-$C_{20}$-Cycloalkenyl-alkyl, $C_4$-$C_{20}$-Alkyl-cycloalkenyl, gegebenenfalls substituiertes $C_9$-$C_{11}$-Bicycloalkyl, $C_{10}$-$C_{15}$-Bicycloalkylalkyl, gegebenenfalls substituiertes $C_{10}$-$C_{15}$-Alkyl-bicycloalkyl,
5 bis 7-gliedriges Heterocycloalkyl mit 1 oder 2 Heteroatomen aus der Gruppe Sauerstoff und/oder Schwefel, 5 bis 7-gliedriges Heterocycloalkylmethyl mit 1 oder 2 Heteroatomen aus der Gruppe Sauerstoff und/oder Schwefel, $C_1$-$C_8$-Alkyl-substituiertes 5 bis 7-gliedriges Heterocycloalkyl oder Heterocycloalkylmethyl mit 1 oder 2 Heteroatomen aus der Gruppe Sauerstoff und/oder Schwefel,
gegebenenfalls substituiertes Aryl, gegebenenfalls substituiertes $C_7$-$C_{20}$-Arylalkyl, $C_7$-$C_{20}$-Alkylaryl, $C_8$-$C_{20}$-Alkyl-aryl-alkyl,

$R^2$ $C_3$-$C_{10}$-Alkyl, $C_3$-$C_8$-Alkoxy,

$R^3$ $C_1$-$C_4$-Alkyl

$R^4$ $C_1$-$C_5$-Alkyl, $C_3$-$C_5$-Alkenyl, $C_3$-$C_5$-Alkinyl und $C_7$-$C_8$-Phenylalkyl,

$X^-$ ein für Pflanzen verträgliches Anion bedeutet,

n den Wert 0 oder 1 hat

oder deren für Pflanzen verträglichen Salzen, behandelt.

5. N-(3,5,5-Trimethyl-hexyl)-3-methyl-4-(p-tert.-butyl-phenyl)-pyrrolidin.

6. N-(3,5,5-Trimethyl-hexyl)-3-methyl-4-(p-tert.-butyl-phenyl)-pyrrolidin.

7. N-(4,4-Dimethyl-pentyl)-3-methyl-4-(p-tert.-butyl-phenyl)-pyrrolidin.

8. N-(Tetrahydropyran-2-yl-methyl)-3-methyl-4-(p-tert.-butyl-phenyl)-pyrrolidin.

9. N-(3,5,5-Trimethyl-hexyl)-N-methyl-3-methyl-4-(p-tert.-butyl-phenyl)-pyrrolidinium-iodid.

10. N-(3,3-Dimethyl-cyclohexyl)-3-methyl-4-(p-tert.-butylphenyl)-pyrrolidin.